# EUROPEAN PATENT SPECIFICATION

(11) **EP 2 849 835 B1**
(45) Date of publication and mention of the grant of the patent: **29.06.2016**
(21) Application number: 13737382.5
(22) Date of filing: 16.05.2013
(51) Int. Cl.: A61M 25/10

(54) **A NON-TEMPORARY CENTRAL VENOUS CATHETER FOR USE IN HAEMODIALYSIS**
NICHTTEMPORÄRER ZENTRALVENÖSER KATHETER ZUR VERWENDUNG IN DER HÄMODIALYSE
CATHÉTER VEINEUX CENTRAL NON TEMPORAIRE DESTINÉ À ÊTRE UTILISÉ EN HÉMODIALYSE

(30) Priority: 16.05.2012 IT FI20120094
(43) Date of publication of application: 25.03.2015
(73) Proprietor: Spindial S.p.A., 43035 Felino (IT); Bandera, Andrea, 38063 Avio (IT)
(72) Inventor: BANDERA, Andrea, I-38063 Avio (IT)
(74) Representative: Valenza, Silvia
(86) International application number: PCT/IB2013/054007
(87) International publication number: WO 2013/171708

(56) References cited:
- EP-A1- 1 374 930
- US-A1- 2006 085 023
- US-A1- 2009 101 577

## Description

### FIELD OF THE INVENTION

The present invention relates to the field of medical and surgical devices and in particular to a long-term or tunnelled Central Venous Catheter (CVC) which can remain in place for long periods of time (months or years). Such a catheter is disclosed in the US 2009 0101577.

### PRIOR ART

The Central Venous Catheter (CVC) is a device which is placed into a large vein in order to carry out haemodialysis treatment. It is used when it is not possible to perform a vascular access using the patient's own vessels or prosthetic vessels.

It is usually made up of a double-lumen cannula, one used for suction and the other enabling re-entry of blood which has been treated and purified using a dialyser.

In some cases we see single-lumen cannulas, and in these cases two separate cannulas must be used. The CVC can be manufactured in various polymers: polyurethane, silicone or copolymers such as carbothane.

CVCs can be temporary, which remain in place for short periods (2-3 weeks) and have immediate percutaneous access through the skin directly to the central vein, or they can be long-term or tunnelled, which can remain in place for much longer periods (months or years) and provide a passage under the skin (subcutaneous tunnel).

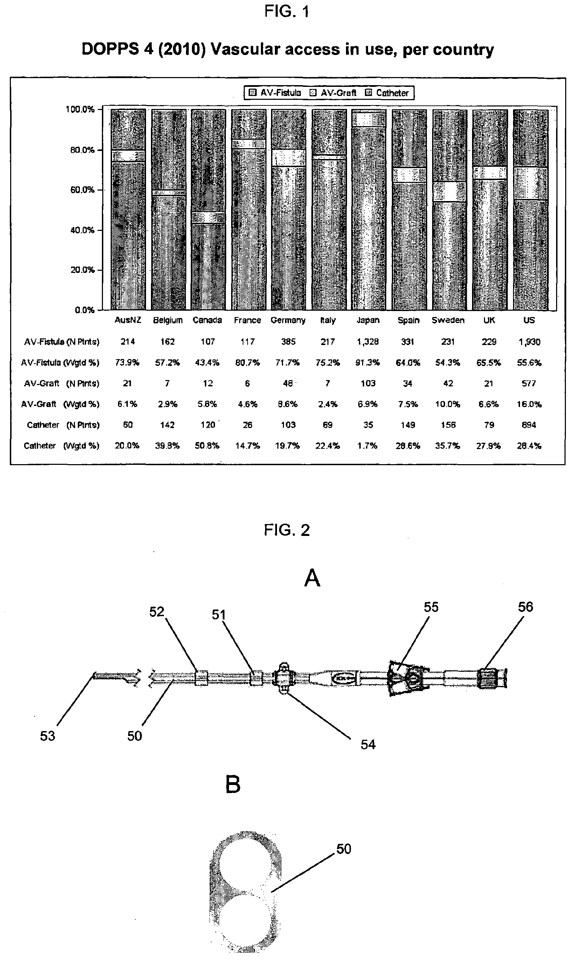

Fig. 1 reports data concerning the vascular access in use in various DOPPS countries (DOPPS 4 data, 2010). The long-term central venous catheter continues to hold a high percentage (10-30% depending on the country) among patients undergoing chronic haemodialysis, despite guideline recommendations to reduce the use of such a device. So far, prevalence trends over the past few years show a continual increase compared with past periods. In Italy, the prevalence of long-term CVCs in patients undergoing haemodialysis was 15% in 2007 (DOPPS 3) and 23.8% in 2010 (DOPPS 4).

The most significant and serious catheter-related complications are infections. Infections related to long-term CVCs are expressed as the following clinical conditions:
- Bacteriaemia or sepsis
- Infection of the exit site and the subcutaneous tunnel

If we compare the various types of vascular access, the relative risk (RR) of access-related bacteraemia with respect to arteriovenous fistula using native veins is 15.5 for patients with a tunnelled CVC and 25.5 for patients with a temporary CVC.

The incidence of infections from long-term (or tunnelled) CVCs is 1.6-5.5 cases/1000 catheter days, and 3.8-6.6 cases/1000 catheter days for temporary catheters.

All tunnelled CVCs which have been marketed and used in clinical practice up until now are featured by a single cuff, usually made from Dacron® and positioned in the end section of the CVC (corresponding to the subcutaneous passage). A few days following positioning, a scarring reaction occurs which anchors the CVC to the subcutaneous tissue and closes the opening preventing micro-organisms from the external environment from entering the bloodstream. You can, however, create these scenarios:
- if the cuff is positioned close to the exit site it provides an excellent barrier against micro-organisms with a low incidence of bacteremia and infections of the subcutaneous tunnel, but a weak anchoring of the CVC with risk of leakage;
- if the cuff is positioned deeper in relation to the subcutaneous tunnel, it provides an excellent anchoring system but a greater stretch of tunnel is exposed to micro-organisms, therefore resulting in a greater risk of infection of the tunnel.

The purpose of this invention is to provide a tunnelled CVC with an improved anchoring system and antibacterial barrier to reduce the incidence of:
1) CVC-related infections
2) subcutaneous tunnel infections
3) displacement of the CVC.

### SUMMARY OF THE INVENTION

The present invention solves the above mentioned problems by way of a non-temporary CVC (50) for use in haemodialysis treatments characterised in that, in the end section to be positioned in the subcutaneous tunnel, it comprises two cuffs (51) and (52) spaced apart by a distance in the range between 3 and 8 cm so that cuff (51) is positioned within the subcutaneous tunnel in the proximity of the exit site (57) and cuff (52) is positioned within the subcutaneous tunnel in the proximity of the access point (58) to the catheterised central vein.

Due to the two cuffs, the CVC according to the invention can be implanted for long periods of time equal to months or years and so can be classified among the so-called long-term or tunnelled CVCs.

Cuff (51), preferably positioned 1-2 cm from the exit site (57), provides an excellent antibacterial barrier whilst cuff (52), preferably positioned 1-2 cm from the point of access (58) to the central vein into which the catheter is inserted, provides excellent anchoring of the CVC to the subcutaneous tissue.

Surprisingly, in preliminary data concerning the use of the new CVC with the double-cuff "Bandera modification" on a sample of 11 patients for a total observation period of 3310 catheter days, only two tunnel infections were observed (corresponding to an incidence of infection equal to 0.6 cases/1000 catheter days) and no displacements.

Therefore, the preliminary results are significant, despite referring to a short observation period and a low number of patients, they show a clear and significant reduction in the incidence of infectious episodes related to the CVC compared to what is reported in the literature (0.6 cases/1000 catheter days vs. 1.6-5.5 cases/1000 catheter days). Such a reduction in the incidence of infections associated with no displacement is an absolutely unexpected result.

The positioning of the CVC has not resulted in any additional difficulty, neither has its removal.

### BRIEF DESCRIPTION OF THE DRAWINGS

FIG 1 shows data concerning the vascular access in use in various DOPPS countries (DOPPS 4 data, 2010);
FIG. 2 (A) shows a bilumen CVC according to the invention equipped with two cuffs (51) and (52); (B) shows a cross-section of the CVC according to the invention.

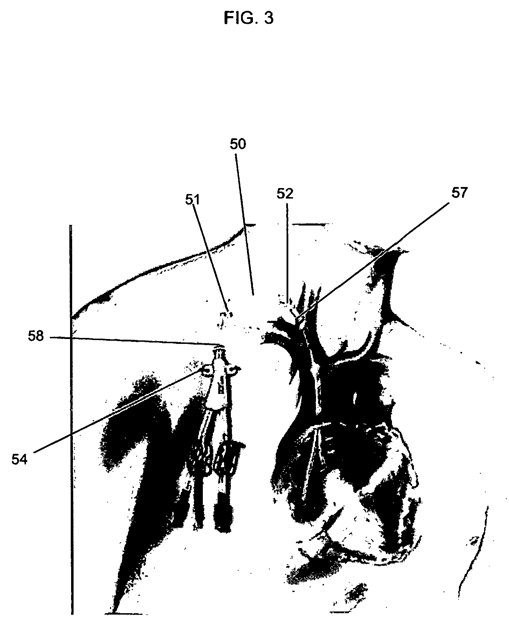

FIG. 3 shows a CVC according to the invention as inserted into a patient who must undergo frequent haemodialysis treatments.

### DETAILED DESCRIPTION OF THE INVENTION

The cuffs (51) and (52) are preferably made of a polyethylene terephthalate fibre (for example DACRON®) or other biocompatible material that causes a fibrotic reaction with the subcutaneous tissue.

The term 'cuff refers to a sleeve (cylindrical tube coaxial to the catheter) applied in a non-sliding manner to the outer surface of the catheter.

The two cuffs (51) and (52) have, independently of each other, a length in the range between 5-10 mm and thickness in the range between 0.5 and 2.0 mm. Preferably, the cuffs have a length of 8 mm and thickness of 1.0 mm. Preferably, the two cuffs (51) and (52) are identical in size and shape.

Preferably, cuff (52) is spaced apart from the tip (53) of the CVC by a distance (x) in the range between 18 and 26 cm. In particular for the right side CVC the distance (x) is preferably in the range between 19 and 20 cm; for the left side CVC the distance (x) is preferably in the range between 23 and 25 cm.

The "double cuff system" can be applied to all types of CVC, both double- and single-lumen.

The material comprising the CVC can be silicone or another material usually used for such medical devices.

Therefore, for example, the CVC according to the invention has, like other known and commercially available CVCs:
- length in the range between 150 and 450 mm,
- external diameter, preferably oval or circular, of between 3.0 and 6.5 mm,
- internal diameter, preferably circular, of between 1.5 and 3.0 mm.

Like other known CVCs, the CVC (50) according to the invention, with reference to Fig. 2A, can preferably be equipped, in a kit, with suture wings (54), one or more clamps (55), one or more luer-lock connectors (56), one or more luer-lock plugs. Below is a table of the preliminary clinical data relating to the positioning of CVCs according to the invention:

| **PATIENT NO.** | **DATE CVC INSERTED** | **TYPE OF CVC** | **DATE OF INFECTION** | **DATE OF REMOVAL** | **Catheter days** |
|---|---|---|---|---|---|
| 1 | 18.02.2010 | BILUMEN | No infection | 02.09.2010 malfunctioning | 196 |
| 2 | 28.07.2009 | BILUMEN | | 05.01.2011 deceased CVC functioning | 526 |
| 3 | 22.07.2009 | BILUMEN | | Deceased 12.11.2010 | 506 |
| 4 | 08.04.2010 | | | Deceased 15.04.2010 | |
| 5 | 12.03.2010 | SINGLE-CANNULA | 01.06.2010 tunnel infection | 22.07.2010 | 130 |
| 6 | 20.11.2009 | BILUMEN | | 19.07.2011 due to death | 590 |
| 7 | 29.06.2010 | BILUMEN | | 01.09.2010 due to death | 60 |
| 8 | 06.07.2010 | TWO SINGLE-CANNULA | | Fully functioning | 590 |
| 9 | 13.07.2010 | TWO SINGLE-CANNULA | 21.09.2010 subcutaneous tunnel infection | 13.01.2011 due to AVF puncture | 184 |
| 10 | 10.08.2010 | Two single-cannula | No infection | 11.11.2010 Showing above the skin | 92 |
| 11 | 19.08.2010 | TWO SINGLE-CANNULA | No infection | 08.11.2011 Due to subcutaneous passage fissure | 440 |

Results of the preliminary study:
- 2 infectious episodes out of a total of 3310 catheter days = 0.6 infectious episodes/1000 CVC days
- no displacements

## Claims

1. A non-temporary central venous catheter (CVC) for use in haemodialysis treatments **characterised in that**, in the end section to be positioned in the subcutaneous tunnel, it comprises two cuffs, a first cuff (51) and a second cuff (52) spaced apart by 3-8 cm so that the first cuff (51) is positioned within the subcutaneous tunnel in the proximity of the exit site (57) of the subcutaneous tunnel and the second cuff (52) is positioned within the subcutaneous tunnel in the proximity of the access point (58) to the catheterised central vein.

2. A CVC according to claim 1, wherein the second cuff (52) is spaced apart from tip (53) of the CVC by a distance (x) in the range between 18 and 26 cm.

3. A CVC according to claim 2, wherein:
- if for the right side, distance (x) is in the range between 19 and 20 cm;
- if for the left side, distance (x) is in the range between 23 and 25 cm.

4. A CVC according to any one of the preceding claims, wherein the two cuffs (51) and (52) are made of polyethylene terephthalate fibre or other biocompatible material that causes a fibrotic reaction with the subcutaneous tissue.

5. A CVC according to any one of the preceding claims, wherein the two cuffs (51) and (52) have, independently of each other, a length in the range between 5-10 mm and thickness in the range between 0.5 and 2.0 mm.

6. A CVC according to claim 4, wherein the cuffs have a length of 8 mm and a thickness of 1.0 mm.

7. A kit comprising a CVC according to any one of the preceding claims.

## Patentansprüche

1. Nicht temporärer zentraler Venenkatheter (CVC) zur Verwendung bei Hämodialysebehandlungen, **dadurch gekennzeichnet, dass** er in dem in dem subkutanen Tunnel zu positionierenden Endabschnitt zwei Manschetten umfasst, eine erste Manschette (51) und eine zweite Manschette (52), die um 3 - 8 cm voneinander beabstandet sind, so dass die erste Manschette (51) in dem subkutanen Tunnel in der Nähe des Austrittsortes (57) des subkutanen Tunnels positioniert ist und die zweite Manschette (52) in dem subkutanen Tunnel in der Nähe des Zugangspunktes (58) zu der katheterisierten Zentralvene positioniert ist.

2. CVC nach Anspruch 1, wobei die zweite Manschette (52) von der Spitze (53) des CVC um eine Distanz (x) im Bereich zwischen 18 und 26 cm beabstandet ist.

3. CVC nach Anspruch 2, wobei:
- wenn für die rechte Seite, die Distanz (x) im Bereich zwischen 19 und 20 cm liegt;
- wenn für die linke Seite, die Distanz (x) im Bereich zwischen 23 und 25 cm liegt.

4. CVC nach einem der vorhergehenden Ansprüche, wobei die beiden Manschetten (51) und (52) aus Polyethylenterephthalatfaser oder einem anderen biokompatiblen Material bestehen, das eine fibrotische Reaktion mit dem subkutanen Gewebe bewirkt.

5. CVC nach einem der vorhergehenden Ansprüche, wobei die beiden Manschetten (51) und (52) unabhängig voneinander eine Länge im Bereich zwischen 5 - 10 mm und eine Dicke im Bereich zwischen 0,5 und 2,0 mm aufweisen.

6. CVC nach Anspruch 4, wobei die Manschetten eine Länge von 8 mm und eine Dicke von 1,0 mm aufweisen.

7. Ausstattung mit einer CVC nach einem der vorhergehenden Ansprüche.

## Revendications

1. Cathéter veineux central longue durée (CVC) destiné à être utilisé dans des traitements d'hémodialyse, **caractérisé en ce qu'**il comprend deux anneaux dans le tronçon d'extrémité devant être positionné dans le tunnel sous-cutané, un premier anneau (51) et un second anneau (52) espacés de 3 à 8 cm, de telle sorte que le premier anneau (51) est positionné à l'intérieur du tunnel sous-cutané à proximité du site de sortie (57) du tunnel sous-cutané, et le second anneau (52) est positionné à l'intérieur du tunnel sous-cutané à proximité du point d'accès (58) à la veine centrale cathétérisée.

2. CVC selon la revendication 1, dans lequel le second anneau (52) est espacé de la pointe (53) du CVC d'une distance (x) comprise entre 18 et 26 cm.

3. (CVC) selon la revendication 2, dans lequel :
- pour le côté droit, la distance (x) est dans la plage de 19 à 20 cm ;
- pour le côté gauche, la distance (x) est dans la plage comprise de 23 à 25 cm.

4. CVC selon l'une quelconque des revendications précédentes, dans lequel les deux anneaux (51) et (52) sont constitués de fibres de polyéthylène téréphtalate ou d'un autre matériau biocompatible provoquant une réaction fibrotique avec le tissu sous-cutané.

5. CVC selon l'une quelconque des revendications précédentes, dans lequel les deux anneaux (51) et (52) présentent, indépendamment l'un de l'autre, une longueur comprise dans la plage de 5 à 10 mm, et une épaisseur comprise dans la plage de 0,5 à 2,0 mm.

6. CVC selon la revendication 4, dans lequel les anneaux ont une longueur de 8 mm et une épaisseur de 1,0 mm.

7. Kit comprenant un CVC selon l'une quelconque des revendications précédentes.
